# EUROPEAN PATENT APPLICATION

(11) **EP 0 594 388 A1**
(43) Date of publication of application: **27.04.1994**
(21) Application number: 93308277.8
(22) Date of filing: 18.10.1993
(51) Int. Cl.: A61B 5/00, A61B 1/04

(54) **Observing device**

(30) Priority: 19.10.1992 JP 279958/92
(71) Applicant: HAMAMATSU PHOTONICS K.K., Shizuoka-ken (JP)
(72) Inventor: Miyakawa, Atsuo, Hamamatsu-shi, Shizuoka-ken (JP); Hirano, Masahiko, c/o Hamamatsu Photonics K. K., Hamamatsu-shi, Shizuoka-ken (JP); Yamashita, Yutaka, c/o Hamamatsu Photonics K. K., Hamamatsu-shi, Shizuoka-ken (JP); Hiruma, Teruo, c/o Hamamatsu Photonics K. K., Hamamatsu-shi, Shizuoka-ken (JP)
(74) Representative: Rackham, Stephen Neil

(57) **Abstract**

Direct optical observations can be made of tissues, cells and the like within a living object by means of an in vivo measuring device (100) including an optical fibre needle (7) formed from a plurality of extremely thin, light transmitting optical fibres (70) bundled into a needle shape. The optical fibre needle (7) can be easily inserted deeply into the object so that its first tip end surface (7eᵢ) contacts a portion to be measured. The first tip end surface (7e₁) receives an optical image which is transmitted toward the second tip end surface (7e₂) of the optical fibre needle (7). An objective lens (1) magnifies the optical image transmitted via the second tip end surface (7e₂) to image pick up means (4).

## Description

The present invention relates to an observation device for observing tissues, cells, and the like deep within a living organism.

There has been known a conventional method in the medical and biological fields for observing tissues, cells, and the like deep within humans, animals, or plants wherein a portion of the living organ or portion of the living plant is removed and prepared into a microscope slide. The sample is magnified and observed with an optical microscope. Another method involves cutting open the area near the portion to be observed, exposing the portion to be observed by, for example, surgical removal, and magnifying the exposed portion with a microscope.

Also, an endoscope is a well-known means of optically observing the physiological condition of the stomach, the intestine, or other similar organ of a person. An endoscope, including a single optical fiber cable, is inserted through the mouth. Optical images introduced into a condenser provided to the tip of the optical fiber cable are optically transmitted outside the body via the optical fiber and picked up by an image pick up device.

However, there has been known a problem with observing a sample removed from an organ with an optical microscope in that samples of organs can not be observed under the same conditions as organs and the like under actual active conditions. For example, even if a sample observed under a microscope is preserved in an activating water solution, obtained observations will probably differ from actual situations in the whole animal because communication is interrupted between the sample and other organs, tissues, and the like that it would normally be connected to. Also, surgically removing other tissues for exposing the portion to be observed can cause side effects so severe that observations become actually impossible.

Although the later described method of inserting an endoscope into a living object does not damage the living body, internal observations are limited to, for example, the innerwall surfaces of organs such as the stomach and the intestine. Observation of portions deep within the walls of these organs at the cellular level have not been possible with this method, particularly because the structure for picking up images of the portion to be observed employs a condenser lens provided at the tip of the sole optical fiber cable. The structure employs optical technology which requires the object and the condenser lens be separated by a distance, that is, the focus is not directly in front of the condenser lens. Therefore, this structure allows observations of open areas in the body, such as the stomach and intestine, but not deep areas. When observations of internal tissues are attempted, tissues directly in front of the lens block the light, preventing obtaining an image of the object at the focus of the lens.

It is therefore an object of the present invention to attempt to solve these conventional problems and provide an in vivo measuring device which can, forex- ample, directly, optically observe tissues, cells and the like deep within living objects.

According to one aspect of the present invention an in vivo observation device for optically observing a portion within a living object comprises:
an optical fibre needle formed from a plurality of light transmitting optical fibres extending in a lengthwise direction and bundled into a needle shape, the optical fibre needle having a first end surface and a second end surface opposed to each other in lengthwise direction for being inserted into a living object to allow the first end surface to confront a portion positioned within the living object, the optical fibre needle receiving, at the first end surface, an optical image of the portion positioned confronting the first end surface and transmitting the optical image toward the second end surface; and
an objective lens provided at a position opposing the second end of the optical fibre needle for receiving and magnifying the optical image transmitted to the second end surface.

The optical fibre needle may receive, at the first end surface, the optical image of the portion positioned in direct contact with the first end surface and transmit the optical image toward the second end surface when the optical fibre needle is inserted into the living object to allow the first end surface to directly contact the portion positioned within the living object.

In the present invention, each of the plurality of light transmitting optical fibers extending in the lengthwise direction is capable of transmitting light so as to allow the optical fiber needle to receive and transmit a two-dimensional optical image of the portion of the living object contacting the first end surface, at a resolution defined dependently on a size of a cross section of each of the light transmitting optical fibers along a plane substantially perpendicular to the lengthwise direction.

The optical fiber needle may preferably have sufficient rigidity for being inserted into the living object without deforming so as to allow the first end surface to directly contact the portion within the living object, the first end surface receiving the two-dimensional optical image generated atthe portion of the living object.

The first end surface of the optical fiber needle may preferably be in the form of a flat surface extending substantially perpendicularly to the lengthwise direction, a flat surface forming a predetermined angle with respect to the lengthwise direction, or a bulging surface.

The in vivo measuring device may further comprise image pick-up means for picking up the two-dimensional optical image magnified by the objective lens and for photoelectrically converting the two-dimensional optical image into an electrical signal. The in vivo measuring device may further comprise display means for receiving the electrical signal produced by the image pick-up means and for reproducing and displaying the two-dimensional optical image based on the electrical signal.

The in vivo measuring device may further comprise: an illumination light source for emitting illumination light; and illumination light guiding means for guiding the illumination light emitted from the illumination light source onto the portion within the living object positioned contacting the first end surface of the optical fiber needle, the two-dimensional optical image being generated at the portion of the living object due to the received illumination light, the optical fiber needle receiving, at the first end surface, the two-dimensional optical image and transmitting the two-dimensional optical image from the first end surface toward the second end surface.

The illumination light guiding means may preferably include selectively guiding means for receiving the illumination light emitted from the illumination light source and the two-dimensional optical image magnified by the objective lens, the selectively guiding means guiding the received illumination light to the objective lens so as to allow the objective lens to guide the illumination light to the second end surface of the optical fiber needle and the optical fiber needle to transmit the illumination light from the second end surface toward the first end surface, the illumination light emitted from the first end surface being radiated on the portion of the living object positioned contacting the first end surface, the selectively guiding means guiding the received two-dimensional optical image to the image pick-up means so as to allow the image pick-up means to pick up the two-dimensional optical image.

According to another aspect, the present invention provides an optically measuring apparatus for optically measuring a portion of an object to be measured, the apparatus comprising: an optical fiber needle formed from a plurality of light transmitting optical fibers extending in a lengthwise direction and bundled into a needle shape, the optical fiber needle having a first end surface and a second end surface opposed to each other in the lengthwise direction, the optical fiber needle receiving, at the first end surface, an optical image of a portion of an object to be measured which is positioned confronting the first end surface and transmitting the optical image toward the second end surface; and an objective lens provided at a position opposing the second end of the optical fiber needle for receiving and magnifying the optical image transmitted to the second end surface.

A particular embodiment of the present invention will now be described with reference to the accompanying drawings, in which:-
Fig. 1 (a) schematically shows an in vivo measuring device of a first preferred embodiment of the present invention;
Fig. 1 (b) is a perspective view of an essential part of an optical assembly of the in vivo measuring apparatus for illustrating how an optical fiber needle is detachably connected to the optical assembly, with a housing member of the optical assembly being partly cut open;
Fig. 2(a) is a sectional side view of an optical fiber needle of the present invention;
Fig. 2(b) is a cross-sectional view of the optical fiber needle taken along a line lib-lib of Fig. 2(a);
Fig. 2(c) is an enlarged cross-sectional view of each optical fiber of the optical fiber needle taken along a line lib-lib of Fig. 2(a);
Fig. 3 schematically shows the manner of measuring a portion deep within a living object with the in vivo measuring device of Fig. 1(a);
Figs. 4 (a) and 4(b) are test results obtained with the in vivo measuring device of Fig. 1 (a) showing two-dimensional images where fluorescent beads were distributed in an object to be measured;
Figs. 5 (a) and 5 (b) are other test results obtained with the in vivo measuring device of Fig. 1(a) showing two-dimensional images of a tissue within a brain of a rat;
Figs. 6 (a) and 6 (b) show modified examples of one tip end surfaces of the optical fiber needle of the present invention; and
Fig. 7 schematically shows an in vivo measuring device of a second embodiment of the present invention.

An in vivo measuring device according to preferred embodiments of the present invention will be described while referring to the accompanying drawings wherein like parts and components are designated by the same reference numerals to avoid duplicating description.

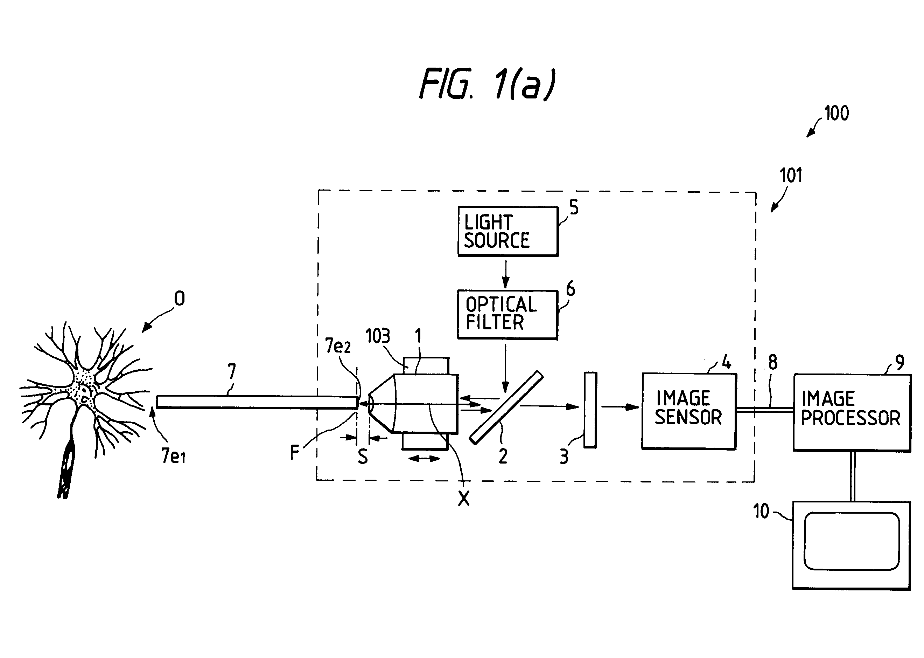

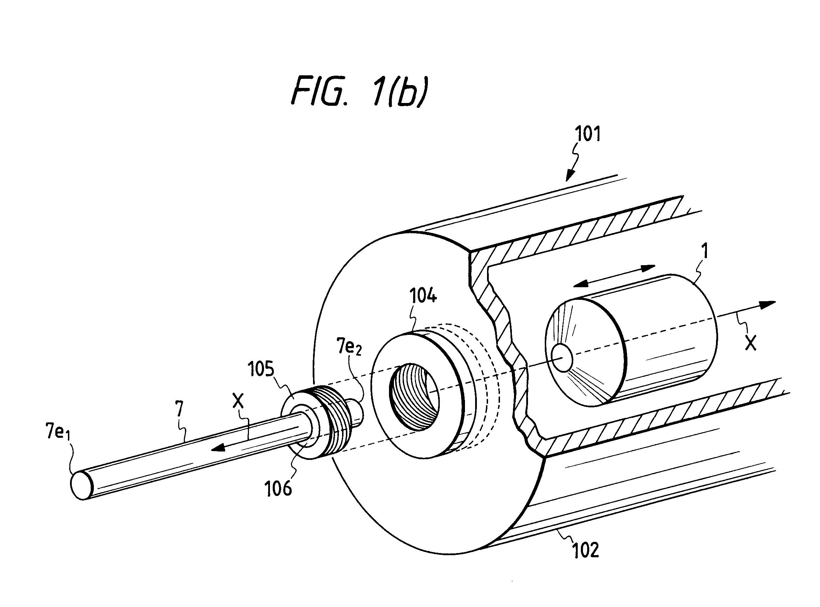

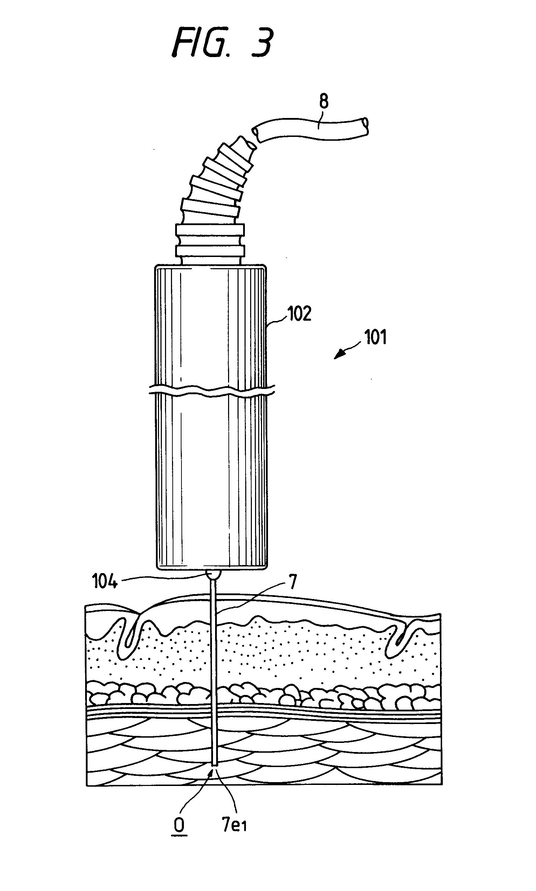

As shown in Fig. 1 (a) and 3, an in vivo measuring apparatus 100 according to a first preferred embodiment of the present invention includes: an optical fiber needle 7 integratedly formed from many extremely thin light transmitting optical fibers bundled together into a needle shape; an optical assembly 101 detachably connected to the optical fiber needle 7; and an image processor 9 and a monitor 10 connected to the optical assembly 101 via a flexible transmission line 8.

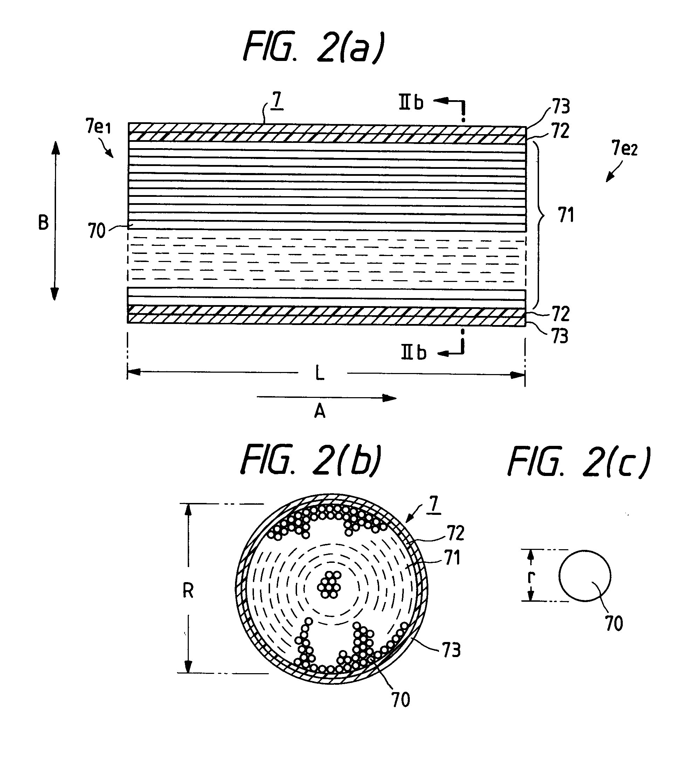

According to the present invention, as shown in the lengthwise sectional side view shown in Fig. 2 (a) and the diametrical cross-sectional view, taken along the line lib-lib in Fig. 2 (a), shown in Fig. 2 (b), the optical fiber needle 7 possesses a structure wherein many extremely thin optical fibers 70 are bundled coaxially into a cylindrical shape with no spaces between the optical fibers. In this embodiment, the optical fiber needle 7 has a length L in the lengthwise direction A of about 5 cm and a total diameter R of about 1 mm. As shown in Fig. 2 (c), the diameter r of each of the thin optical fibers 70 is about 3 µm. About one hundred thousand (100000) optical fibers 70 form an integrated structure in this approximately 1 mm diameter.

To produce the optical fiber needle 7, at first, a certain number of optical fibers 70 made of glass are bundled, drawn out and stretched in the lengthwise direction Awhile being heated. Then, a plurality of optical fiber bundles stretched in this way are again bundled and further stretched in the lengthwise direction Awhile being heated. This process is repeated to form an integrated optical fiber bundle 71 with no gaps between individual optical fibers 70. A thin shield layer 72 is provided for covering a peripheral surface of the optical fiber bundle 71 to prevent external light from entering through the peripheral surface thereof. For reinforcing strength and to further prevent external light from entering through the peripheral surface of the optical fiber bundle 71, the optical fiber bundle 71 covered with the shield layer 72 is then inserted in and integrated with a rigid, thin, hollow cylinder 73 made of, for example, metal. Thus produced optical fiber needle 7 has opposite tip end surfaces 7eᵢ and 7e₂ in the lengthwise direction A. Each of the tip end surfaces 7eᵢ and 7e₂ is at right angles to the lengthwise direction. Thus, the optical fiber needle 7 is formed in a needle shape.

The optical fiber needle 7 has a diameter of about 1 mm, sufficiently small to be inserted into a living body without causing excessive damage. The optical fiber needle 7 is reinforced by the rigid cylinder 73 so as to be sufficiently rigid to be inserted into the living body without deforming. Thus, the optical fiber needle 7 can be directly inserted into a living body for observations. As will be described in detail later, the optical fiber needle 7 is inserted into a living object at the tip end 7eᵢ. In the optical fiber needle 7, a plurality of optical fibers 70 are two-dimensionally arranged along a plane B orthogonal to the lengthwise direction A. The optical fiber bundle can therefore receive a two-dimensional optical image at the tip end 7eᵢ, transmit the two-dimensional optical image to the tip end 7e₂ without deforming it, and emit the two-dimensional optical image from the tip end 7e₂. The diameter r of the individual optical fibers 70 therefore determines the resolution of the device for picking up the optical image. According to the present invention, the extremely small diameter r of the optical fibers 70 of about 3 µm provides a high resolution, two-dimensional image, allowing discernment of individual cells or the like distributed in the living body.

As shown in Figs. 1(a), 1 (b) and 3, the optical fiber needle 7 is detachably connected to the optical assembly 101 (designated by the dotted line in Fig. 1 (a)). The optical assembly 101 includes: an objective lens 1 having an optical axis X; a dichroic mirror 2 positioned behind the objective lens 1 in optical axial alignment therewith: an interference filter 3 positioned behind the dichromatic mirror 2 in optical axial alignment therewith; a two-dimensional image sensor 4, for example, a charge-coupled device (CCD), positioned behind the interference filter 3; a light source 5 for emitting light; and an optical filter 6 for receiving light emitted from the light source and separating a desired wavelength of light h from the received light, the optical filter being positioned in optical axial alignmentwith the dichromatic mirror2. The optical assembly 101 has a cylindrical housing member 102 within which the light source 5 and the optical filter 6 are fixedly mounted. The dichroic mirror 2 and the interference filter 3 are detachably mounted within the housing member 102. The objective lens 1 is fixedly mounted on a one-dimensional stage 103 which is mounted within the housing member 102 to be movable in the optical axial direction X of the objective lens 1.

As shown in detail in Fig. 3, a needle mounting member 104 is fixedly secured to a top or front wall of the cylindrical housing member 102 at a predetermined position confronting the objective lens 1 in optical axial alignment therewith. The needle mounting member 104 has a threaded through-hole. When detachably connecting the optical fiber needle 7 to the optical assembly 101, the optical fiber needle 7 is first inserted, via a rubber member 106, into a ring member 105 at its portion adjacent to the tip end 7e₂. The ring member 105 has a threaded outer peripheral wall. Screwing the ring member 105 into the through-hole of the needle mounting member 104 fixedly secures the optical fiber needle 7 to the mounting member 104. Thus, the optical fiber needle 7 is positioned in front of the objective lens 1 in optical axial alignment therewith. In this manner, the optical fiber needle 7 can be detachably connected to the optical assembly 101. Moving the one-dimensional stage 103 toward and away from the optical fiber needle 7 thus fixedly secured to the needle mounting member 104 adjusts a distance S between the tip end surface 7e₂ and the objective lens 1 to a value equal to a focal length of the objective lens 1. Thus, moving the one-dimensional stage 103 positions the objective lens 1 so that the focal plane F of the objective lens 1 may coincide with the tip end surface 7e₂.

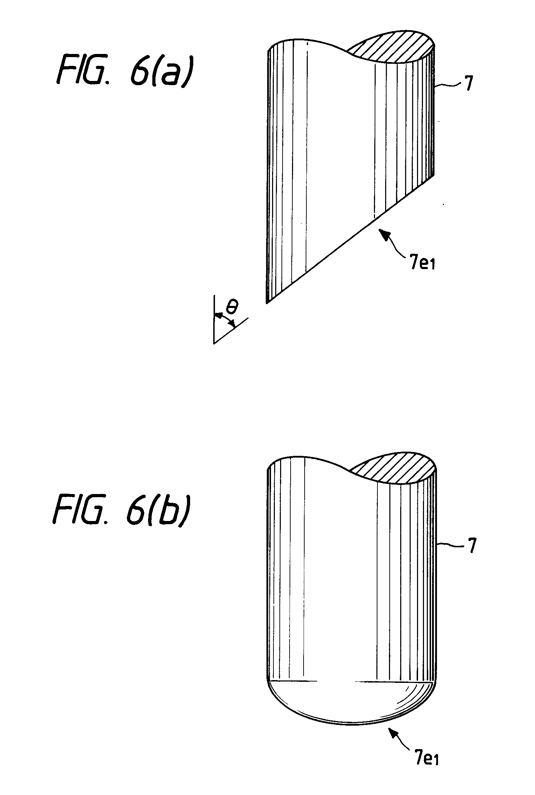

When the optical fiber needle 7 is thus connected to the optical assembly 101, the objective lens 1 confronts the tip end 7e₂. Accordingly, the tip end 7eᵢ can be directly inserted into the living object and directly face or contact a portion of the living object (object portion O) to be measured. It therefore becomes possible to observe the object portion while in direct contact therewith. In addition, since the objective lens is not positioned confronting the tip end 7eᵢ, the tip end 7eᵢ of the optical fiber needle 7 may be produced to have a small diameter sufficient to be inserted into the living object. In addition, the tip end 7eᵢ may be easily processed into shapes desired to be inserted into the living body organ. The tip end 7e₁ may be processed not only to the right angle as shown in Fig. 2(a) but also to various other shapes. For example, the tip end 7e₁ may be processed or polished to an angle 0 as shown in Fig. 6 (a). The angle 0 may preferably be acute. The tip end 7e₁ may also be processed or polished to a bulging spherical tip as shown in Fig. 6 (b).

The light source 5 includes, for example, a xenon lamp with luminary output of 75 W. The optical filter 6 is provided with a plurality of optical band pass filters through which can pass different wavelength lights. By an operator switching these optical band-pass filters and setting one of the band-pass filters on an optical path from the light source 5, a specific wavelength light hν can be selectively irradiated onto the dichroic mirror 2. According to one illustrative example of the present invention, the optical filter 6 may be operated to select such an optical band-pass filter through which can pass light having a predetermined wavelength λ₁. The predetermined wavelength excites cells or other components distributed in the object portion O and causes the cells or other components to emit light, such as fluorescent light, having another predetermined wavelength X₂ higher than the wavelength λ₁. The light of the wavelength λ₁ will be referred to as an "excitation or illumination light," hereinafter, and the light of the wavelength X₂ will be referred to as an "emission light," hereinafter.

It is noted that the tight from the optical filter can be introduced to the dichroic mirror using optical fibers. In this case, the light source 5 and the optical filter 6 can be separated from the optical assembly 101.

The dichroic mirror 2 produced with a boundary at wavelength λb can transmit almost 100% of light with wavelength equal to or greater than the wavelength λb but reflect almost 100% of light with wavelength less than the wavelength λb. Accordingly, in the illustrative example, the boundary wavelength λb of a dichroic mirror mounted in the optical assembly 101 should be higher than the wavelength λ₁ and equal to or lower than the wavelength λ₂. Excitation light from the optical filter 6 having wavelength λ₁ reflects off the dichroic mirror2, passes through the objective lens 1 into the individual optical fibers 70 at the tip 7e₂ of the optical fiber needle 7, illuminating at the tip 7e₁ the object portion O such as a tissue or cells within a living body. The cells or other components distributed in the object portion O are excited by the excitation light of wavelength λ₁ to emit emission light, such as fluorescent light, of wavelength λ₂. The tip end 7e₁ directly contacting the object portion O receives a two-dimensional optical image on which the thus generated emission light is distributed. This optical image represents the distribution state of the cells or other components distributed in the object portion O. The optical fiber needle 7 can transmit the optical image from the tip end 7e₁ toward the tip end 7e₂, from which the optical image is emitted.

As described previously, the objective lens 1 is located so that its focal plane is positioned at t he tip end surface 7e₂. Accordingly, the optical image emitted from the tip end surface 7e₂ is magnified with the objective lens 1 at a predetermined magnification. The dichroic mirror2 receives the magnified optical image and transmits it, because the wavelength X₂ of the optical image is equal to or higher than the boundary wavelength λb.

The interference filter 3 is provided for receiving the optical image transmitted through the dichroic mirror 2 and for clarifying the optical image before emitting it. An interference filter 3 of a type capable of transmitting only light of the wavelength λ₂ and improving the optical image of the wavelength λ₂ should be selected to be mounted in the housing member 102.

As described above, the single optical fiber needle 7 functions both to transmit the excitation light from the light source 5 to the object portion O and to transmit the emission light emitted from the object portion O to the interference filter 3. The dichroic mirror 2 separates the excitation light to be illuminated on the object portion O and the emission light emitted from the object portion O so that both can be transmitted through the single optical fiber needle 7.

The two-dimensional image sensor 4 is provided for dynamically picking up the optical image from the interference filter 3 at its image receiving surface at synchronous timing based on a standard television format, such as a National Television System Committee (NTSC) format. The image sensor4 photoelectrically converts the optical image into an electrical image. The image sensor 4 outputs electrical image signals representative of the electrical image to the image processor 9 via the transmission line 8 made from, for example, a coaxial cable. The image processor 9 is an internal microcomputer system having various calculation functions for performing signal processes (contrast enhancement and noise reduction, etc.) on the inputted electrical image signals to improve the image. The image processor 9 further possesses a function for, for example, recording the processed electrical image signals on a magnetic recording medium, etc. The image processor converts the processed electrical image signals into television signals based on the standard television format, and supplies the television signals to an external monitor 10. The monitor 10 possesses functions capable of, for example, reproducing the dynamically picked up optical image of the object portion O in real time, reproducing an image based on the electrical image signals temporarily recorded on the magnetic recording medium, etc., and still framing the reproduced image. Power for driving the light source 5, the two dimensional image sensor 4, and the optical filter 6 in the optical assembly 101 is supplied from an external power source via an electric wire (not shown) integrally provided to and following along, for example, the transmission wire 8 for transmitting the electrical image signals.

Next, the operations of the in vivo measuring device 100 of the present embodiment will be explained. First, as shown in Fig. 3, an operator grasps the housing 102 and inserts the tip end 7eᵢ deeply into the living object, in the same manner as inserting a syringe needle, until the tip end surface 7eᵢ of the optical fiber needle 7 opposes and touches the portion O of the object such as a tissue or an organ in a living body.

Then, the operator turns on a switch (not shown in the drawing) mounted on the housing 102 of the optical assembly so that power is supplied to the various elements in the optical assembly 101. As a result, the light of the wavelength λ₁, incident via the light source 5, the optical filter 6, the dichroic mirror 2, and the objective lens 1, illuminates the object portion O as excitation light emitted from the tip end surface 7eᵢ. The two-dimensional optical image of the object portion O obtained by this illumination is incident on the tip end surface 7eᵢ and transmitted to the tip end surface 7e₂ (opposing the objective lens 1). The objective lens 1 magnifies the optical image from the tip end surface 7e₂ at a predetermined magnification and transmits it to the dichroic mirror 2. The dichroic mirror 2 transmits the magnified optical image to the image receiving surface of the two-dimensional image sensor 4 via the interference filter 3. The two-dimensional image sensor4 converts the optical image into electrical image signals synchronized at the predetermined synchronization timing, and transmits the electrical image signals to the signal processor 9. The monitor 10 reproduces the optical image of the object portion.

It is noted that the reason why the optical fiber needle 7 is processed into a thin, needle shape is to reduce possibilities of injuring the living persons, animals or plants under study by inserting the optical fiber needle 7. The present invention allows observations at the cellular level of tissues, deep within the living body of an animal, that cannot be viewed from the surface. Measurements are possible of physiological activity. By performing this operation while observing the monitor 10, the object portion O to be measured can be accurately detected.

### FIRST TEST

The present inventors conducted a first series of measurements for estimating the spatial resolution of the device according to the present embodiment and the transparency of the optical fiber needle 7. In this first series of measurements, the present inventors observed two types of tiny fluorescent beads B1 and B2.

In the first measurement, fluorescent beads B1 with mean diameter of 9.4 µm were coated with a fluorescent dye which emits fluorescent light of wavelength of 520 nm or more when excited with blue light (excitation maximum, 458 nm). To observe the beads B1, the optical filter 6 was operated to set a 450 nm wavelength optical band-pass filter in an optical path from the light source 5. A dichroic mirror 2 with a boundary wavelength of 510 nm was used. As the two-dimensional image sensor4, a simple solid imaging device (CCD) was used. The beads B1 fluoresced a 520 nm or more light upon being illuminated with 450 nm by such a set up in the in vivo measuring device 100. The CCD 4 picked up a two-dimensional optical image in which fluorescence of wavelength of 520 nm or more was distributed in a pattern equaling the distribution of the fluorescent beads B1. The image was then reproduced on the monitor 10 based on NTSC television format, and printed on a silver film at the resolution of the monitor.

In the second measurement, fluorescent beads B2 with mean diameter of 3.41 µm were coated with a fluorescent dye which emits fluorescent light of wavelength of455 nm or more upon excited at ultraviolet light (excitation maximum, 365 nm). In this case, the optical filter 6 was operated to set a 380 nm wavelength optical band-pass filter. The dichroic mirror 2 with a boundary wavelength of 455 nm was used. The fluorescent beads B2 fluoresced a 455 nm or more light when illuminated at 380 nm. The resultant two-dimensional image picked up by the CCD 4 was then reproduced on the monitor 10 based on NTSC television format, and printed on a silver film at the resolution of the monitor.

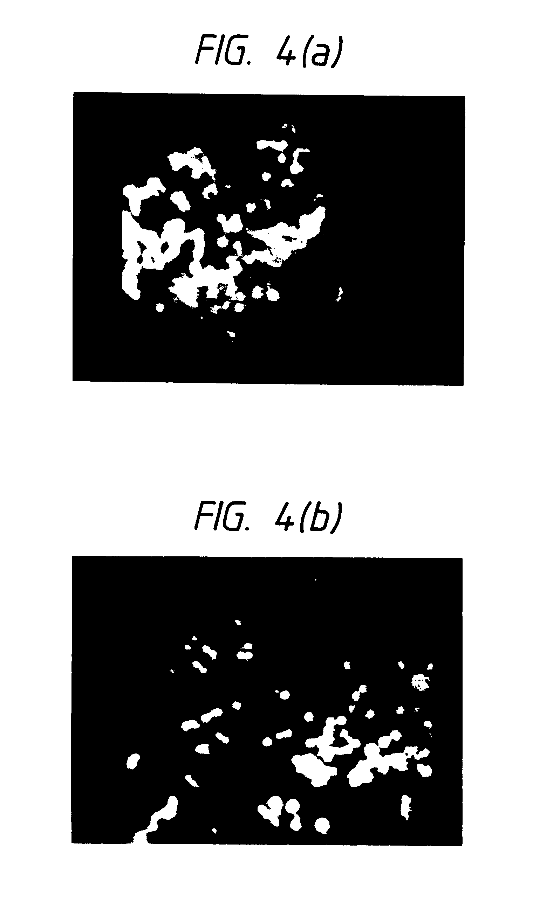

Figs. 4 (a) and 4 (b) show images thus obtained for the beads B1 and B2, respectively. The distribution pattern of the fluorescent beads are clearly imaged with high contrast. These experimental results show that even if a simple solid imaging device such as a CCD is used as the two-dimensional image sensor 4, a high quality image can be obtained. By using such a simple solid imaging device, the entire size of the device can be reduced.

To illuminate the beads B2 with light of wavelength shorter than 380 nm, the present inventors further conducted another measurement in which an optical band-pass filter which can transmit light of wavelength shorter than 380 nm was set in the optical path of light from the light source 5. During excitation with light of wavelength shorter than 380 nm, however, the fluorescence of the beads could not be observed clearly. It can be thought that this was caused by limitations in the transparency of the optical fiber needle 7 for ultraviolet wavelengths. When measurements are to be performed with weak light such as ultraviolet light, therefore, an image intensifier may preferably be used as the two-dimensional image sensor 4.

### SECOND TEST

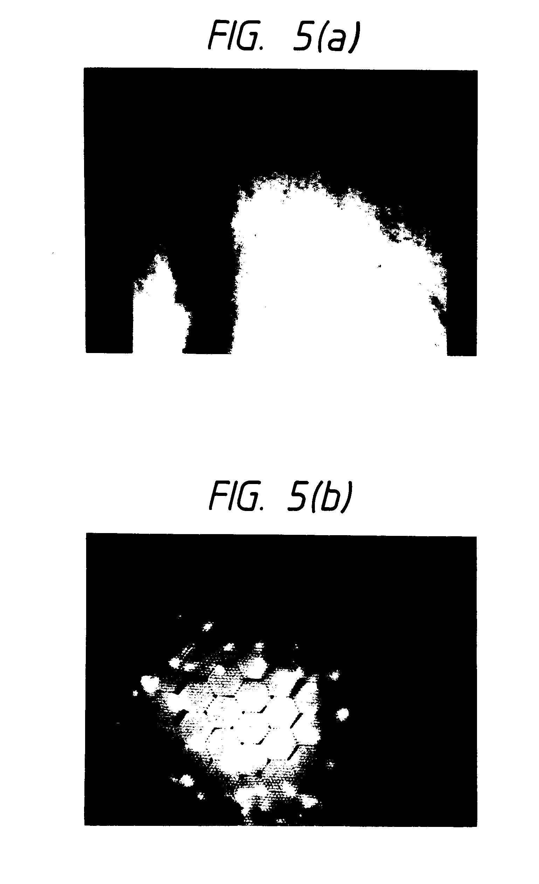

The present inventors conducted a second series of measurements to observe the brain tissue of an anesthetized rat. The present inventors inserted the optical fiber needle 7, in the manner shown in Fig. 3, deep into the brain of the anesthetized rat through a hole opened in the skull thereof. Brain cells generally include compound which fluoresces 400 nm or more wavelength light when excited with ultraviolet light (which will be referred to as "fluorescent compound," hereinafter). The optical filter 6 was operated to set a 380 nm wavelength optical band-pass filter. The dichroic mirror 2 with a boundary wavelength of 400 nm was used. The brain cells fluoresced 400 nm or more light when illuminated at 380 nm. The resultant two-dimensional image picked up by the CCD 4 was then reproduced on the monitor 10 based on NTSC television format, and printed on a silver film at the resolution of the monitor. Fig. 5 (a) shows the thus obtained print image. Since only a small amount of the fluorescent compound is found in blood vessels, the black area appearing at the upper left of Fig. 5 (a) is obviously a blood vessel.

In a second measurement, the present inventors stained the brain cellswith a fluorescent dye (acridine orange dye). Acridine orange dye fluoresces 455 nm or more wavelength light when excited at ultraviolet light (excitation maximum, 365 nm). The optical filter 6 was operated to set a 380 nm wavelength optical band-pass filter. The dichroic mirror 2 with a boundary wavelength of 455 nm was used. The brain cells fluoresced 455 nm or more light when illuminated at 380 nm. The resultant two-dimensional image picked up by the CCD 4 was then reproduced on the monitor 10 based on NTSC television format, and printed on a silver film at the resolution of the monitor. Fig. 5 (b) shows the print obtained for this observation. Each of the fine dots systematically aligned in this diagram is an image produced with an individual optical fiber with radius of 3 f..lm. Images of individual dyed cells are observable in the diagram as white spots, several times larger than, and overlapping, the systematically aligned fine dots.

As can be clearly seen from these test results, subjective measurements can be made while the living object is in an active state without damaging the internal tissue and the like of the living object. Particularly, by staining with a suitable fluorescent dye, individual cells can be more clearly measured. The test results therefore confirm that the in vivo measuring device 100 of the present embodiment can measure the object portion O at single cell level.

In recent years, a reagent has been developed for measuring pH, calcium ion density, and the like in a cell from the change in fluorescent intensity. There has been known a technology for measuring cell activity by using this reagent. This reagent is conventionally known to be suitable for cultivated cells, tissue sections, and the like. However, by combining the in vivo measuring device of this embodiment with this reagent, various tissues, cells, and the like can be measured while the living object is performing activities.

The effects of the present invention can be applied to many situations, including observing conditions of tissues, cells, and the like of, for example, areas deep in the chest without opening the chest by surgery, preparatorily observing tissues within a living object before surgery, and directly observing diseased tissues instead of performing biopsy collections for forming pathologic samples in clinical tests.

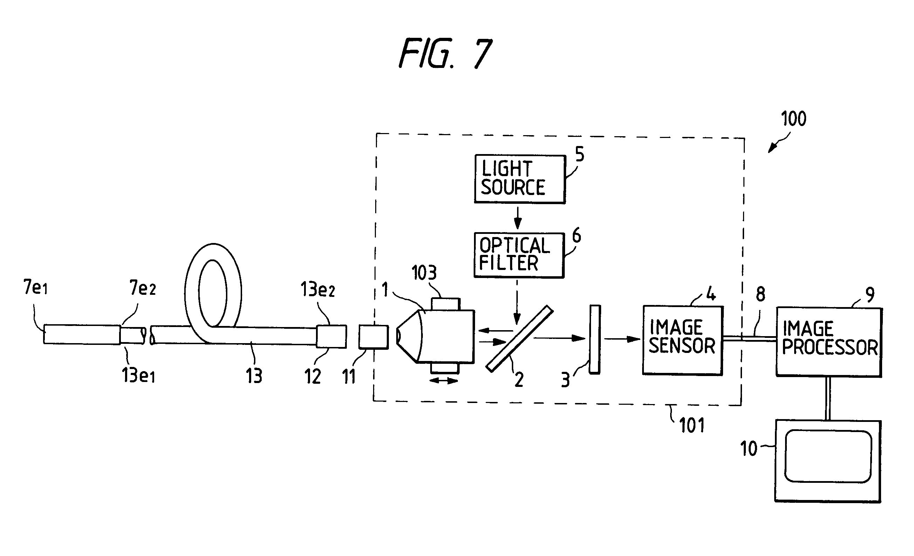

Next, a second preferred embodiment according to the present invention will be explained while referring to Fig. 7. Essential components of this embodiment are substantially the same as those in the embodiment shown in Fig. 1(a), with the exception that the optical fiber needle 7 is indirectly connected to the optical assembly 101. Similar components of Fig. 1(a) and Fig. 7 are provided with the same numbering.

According to the second embodiment, the optical fiber needle 7 is combined with a flexible optical transmission cable 13 into an optical probe. More specifically, the optical fiber needle 7 is fixedly secured, at its tip end 7e₂, to one end 13eᵢ of an optical transmission cable 13. The optical transmission cable 13 has almost the same structure as the optical fiber needle 7 with the exception that the optical transmission cable 13 is very long and not covered with the rigid cylinder73. Accordingly, the optical transmission cable 13 is a long and flexible optical fiber bundle formed from a plurality of long, extremely thin optical fibers. The optical transmission cable 13 has the diameter of about 1 mm and each of the extremely thin optical fibers forming the optical transmission cable 13 has the small diameter of about 3 f..lm.

In the optical assembly 101 of this embodiment, as shown in Fig. 7, an optical connector 11 is fixedly secured to the front or top wall ofthe housing member 102 at a position confronting the objective lens 1 in optical alignment therewith. The optical connector 11 may be in the same form as that of the needle mounting member 104 of Fig. 1(b). An optical connector 12 is fixedly secured to a tip end 13e₂ of the optical transmission cable 13 which is opposed to the tip end 13eᵢ at which the transmission cable 13 is fixed to the optical fiber needle 7. When detachably connecting the transmission cable 13 to the optical assembly 101, the optical connector 12 is detachably fitted to the optical connector 11. The optical connector 12 may be in the same form as that of the ring member 105 of Fig. 1(b). It is noted that in the present embodiment, the objective lens 1 is positioned so that the focal plane of the objective lens coincides with the tip end 13e₂ of the transmission cable 13.

After connecting the optical connectors 11 and 12 together, the operator grasps the optical fiber needle 7 and inserts it deeply into a living object, while the optical assembly 101 is placed on a table. When the tip end 7eᵢ of the needle 7 contacts the object portion O to be measured, measurements start. That is, light generated at the light source 5 within the optical assembly 101 is transmitted through the transmission cable 13 and the needle 7 to illuminate the object portion O. An optical image of the object portion O incident to the tip end 7eᵢ is then transmitted in the same optical path (the needle 7 and the cable 13) but in the reverse direction, to be emitted to the objective lens 1.

According to the present embodiment, because the optical transmission cable 13 is formed from the flexible fiber bundle, optical image transmission is not obstructed by, for example, bending the cable. Because the optical transmission cable 13 is formed from the extremely thin optical fibers which have diameters equal to those of the optical fiber needle 7, the optical transmission cable 13 can transmit the optical image inputted from the optical fiber needle 7 without deteriorating resolution of the optical image. Furthermore, according to this embodiment, it becomes unnecessary to lift up and grasp the comparatively large optical assembly 101. The operator can directly grasp and operate the needle 7 and insert it into the object to be measured. The flexible transmission cable 13 can follow the operator's manipulation of the needle 7. The flexible transmission cable 13 therefore allows greater ease of operation.

As described above, the present invention uses an optical fiber needle formed from a plurality of extremely thin, light transmitting optical fibers bundled into a needle shape. The optical fiber needle can be easily inserted deeply into an object so that its first tip end surface confronts or contacts a portion to be measured which is positioned deeply within the object. The first tip end surface receives an optical image of the portion to be measured. The optical fiber needle transmits the optical image from the first tip end surface toward its second tip end surface which is positioned opposite to the first tip end surface. An objective lens is provided opposing the second tip end surface of the optical fiber needle for receiving and magnifying the optical image transmitted to the second tip end surface.

Preferably, the first tip end surface of the optical fiber needle may be formed to either an angled or a bulging shape to improve ease of insertion into the living object.

Still further, light may be inputted, from the objective lens to the second tip end surface of the optical fiber needle, for illuminating the object portion to be measured.

With such a structure, the object portion such as a tissue or cells deep in a living organism can be measured via the objective lens. Even if the object portion to be measured is concealed from the objective lens, the optical fiber needle allows measurements equivalent to those performed when the object is actually exposed. Therefore, measurement within a living body, which conventionally are considered extremely difficulty, can be performed without damaging the living object.

While the invention has been described in detail with reference to specific embodiments thereof, it would be apparent to those skilled in the art that various changes and modifications may be made therein.

For example, although the objective for developing the in vivo measuring device according to the present invention is mainly to allow observations and the like of tissues and cells in an actively living body without damaging the living body, the present invention is also suitable for various conventional uses, such as observing internal sections of organs and the like that have been removed from the body.

In the above-described embodiment, an attachment/detachment assembly (104 and 105) fordetach- ably connecting the optical fiber needle 7 to the optical assembly 101 is in the form of a screw assembly for screwing the optical fiber needle 7 into the optical assembly 101. However, this attachment/detachment assembly may also be in the form of a bayonet assembly for engaging the optical fiber needle 7 with the optical assembly 101. The structure of the attachment/detachment assembly may be freely selected.

In the above-described illustrative example, the optical filter is operated to irradiate the object portion with excitation light of wavelength Â₁ and to cause the object portion to fluoresce emission light of wavelength X2 which is higher than the wavelength Â1. Accordingly, the dichroic mirror 2 of a boundary wavelength λb (λ₁ < λ_{b} ≦ X₂) is used to separate the excitation light and the emission light. However, according to the present invention, other various types of illumination light can be used for causing the object portion to generate emission light. Various types of means can be used for separating the illumination light and the emission light which are transmitted through the single optical fiber needle.

## Claims

1. An in vivo observation device for optically observing a portion within a living object, said in vivo measuring device comprising:
an optical fibre needle formed from a plurality of light transmitting optical fibres extending in a lengthwise direction and bundled into a needle shape, the optical fibre needle having a first end surface and a second end surface opposed to each other in lengthwise direction for being inserted into a living object to allow the first end surface to confront a portion positioned within the living object, the optical fibre needle receiving, at the first end surface, an optical image of the portion positioned confronting the first end surface and transmitting the optical image toward the second end surface; and
an objective lens provided at a position opposing the second end of the optical fibre needle for receiving and magnifying the optical image transmitted to the second end surface.

2. The in vivo observation device as claimed in claim 1, wherein said optical fibre needle receives, at the first end surface, the optical image of the portion positioned in direct contact with the first end surface and transmits the optical image toward the second end surface when said optical fibre needle is inserted into the living object to allow the first end surface to directly contact the portion positioned within the living object.

3. The in vivo observation device as claimed in claim 2, wherein each of the plurality of light transmitting optical fibres extending in the lengthwise direction is capable of transmitting light so as to allow said optical fibre needle to receive and transmit a two dimensional optical image of the portion of the living object contacting the first end surface, at a resolution defined dependently on a size of a cross section of each of the light transmitting optical fibres along a plane substantially perpendicular to the lengthwise direction.

4. The in vivo observation device as claimed in any preceding claim, wherein said optical fibre needle has sufficient rigidity for being inserted into the living object without deforming so as to allow the first end surface to directly contact the portion within the living object, the first end surface receiving the two dimensional optical image generated at the portion of the living object.

5. The in vivo observation device as claimed in claim 4, wherein said optical fibre needle includes a reinforcing member for reinforcing said optical fibre needle so as to allow said optical fibre needle to have a sufficient rigidity to be inserted into the living object without deforming.

6. The in vivo observation device as claimed in claim 5, wherein said optical fibre needle includes:
a bundle of the plurality of light transmitting optical fibres; and
a rigid hollow member provided around the periphery of the bundle.

7. The in vivo observation device as claimed in any preceding claim, wherein the first end surface of said optical fibre needle includes a flat surface extending substantially perpendicularly to the lengthwise direction, or forms a pre-determined angle with respect to the lengthwise direction, or includes a bulging surface.

8. The in vivo observation device as claimed in any preceding claim, further comprising image pick up means for picking up the two dimensional optical image magnified by the objective lens and for photo-electrically converting the two dimensional optical image into an electrical signal.

9. The in vivo observation device as claimed in claim 8, further comprising display means for receiving the electrical signal produced by said image pick up means and for reproducing and displaying the two dimensional optical image based on the electrical signal.

10. The in vivo observation device as claimed in any preceding claim, further comprising:
an illumination light source for emitting illumination light; and
illumination light guiding means for guiding the illumination light emitted from said illumination light source onto the portion within the living object positioned contacting the first end surface of said optical fibre needle, the two dimensional optical image being generated at the portion of the living object due to the received illumination light, said optical fibre needle receiving, at the first end surface, the two dimensional optical image and transmitting the two dimensional optical image from the first end surface toward the second end surface.

11. The in vivo observation device as claimed in claim 10, wherein said illumination light guiding means guides the illumination light emitted from said illumination light source to the second end surface of said optical fibre needle, said optical fibre needle transmitting the illumination light from the second end surface toward the first end surface, the illumination light emitted from the first end surface being radiated on the portion of the living object contacting the first end surface.

12. The in vivo observation device as claimed in claim 11, further comprising separating means for separating the two dimensional optical image transmitted to the second end surface through said optical fibre needle and the illumination light transmitted to the second end surface by said illumination light guiding means.

13. The in vivo observation device as claimed in claim 8 or any one of claims 9 to 12 when dependent upon claim 8, wherein said illumination light guiding means includes selectively guiding means for receiving the illumination light emitted from said illumination light source and the two dimensional optical image magnified by said objective lens, said selectively guiding means guiding the received illumination light to said objective lens so as to allow said objective lens to guide the illumination light to the second end surface of said optical fibre needle and said optical fibre needle to transmit the illumination light from the second end surface toward the first end surface, the illumination light emitted from the first end surface being radiated on the portion of the living object positioned contacting the first end surface, said selectively guiding means guiding the received two dimensional optical image to said image pick up means so as to allow said image pick up means to pick up the two dimensional optical image.

14. The in vivo observation device as claimed in claim 13, wherein the illumination light has a first wavelength and the two dimensional optical image has a second wavelength different from the first wavelength, and wherein said selectively guiding means includes a dichroic mirror for reflecting off the illumination light of the first wavelength to guide the illumination light to said objective lens and for transmitting the two dimensional optical image of the second wavelength to guide the two dimensional optical image to said image pick up means.

15. The in vivo observation device as claimed in any preceding claim, wherein the second end surface of said optical fibre needle is positioned on a focal plane of said objective lens so as to allow said objective lens to magnify the two dimensional optical image transmitted to the second end surface at a pre-determined magnification.

16. The in vivo observation device as claimed in any preceding claim, further comprising an optical fibre cable formed from a plurality of light transmitting optical fibres extending in a lengthwise direction, said optical fibre cable having a third end surface and a fourth end surface opposed to each other in its lengthwise direction, the third end surface of said optical fibre cable being optically connected to the second end surface of said optical fibre needle for receiving the two dimensional optical image transmitted to the second end surface, said optical fibre cable transmitting the received two dimensional optical image from the third end surface to the fourth end surface, said objective lens being provided at a position opposing the fourth end surface of said optical fibre cable for receiving and magnifying the two dimensional optical image transmitted to the fourth end surface.

17. The in vivo observation device as claimed in claim 18, wherein the fourth end surface of said optical fibre cable is positioned on a focal plane of said objective lens so as to allow said objective lens to magnify the two dimensional optical image transmitted to the fourth end surface at a pre-determined magnification.
